(19)

Europäisches
Patentamt
European
Patent Office
Office européen
des brevets

(11)  **EP 4 723 129 A1**

(12)  **EUROPEAN PATENT APPLICATION**

(43) Date of publication:
  **08.04.2026   Bulletin 2026/15**

(51) International Patent Classification (IPC):
  **G16H 70/40** (2018.01)     G16H 20/10 (2018.01)
  **G16H 50/50** (2018.01)

(21) Application number: **25201958.3**

(22) Date of filing: **12.09.2025**

(52) Cooperative Patent Classification (CPC):
  **G16H 70/40**; G16H 20/10; G16H 50/50

(84) Designated Contracting States:
  **AL AT BE BG CH CY CZ DE DK EE ES FI FR GB
  GR HR HU IE IS IT LI LT LU LV MC ME MK MT NL
  NO PL PT RO RS SE SI SK SM TR**
  Designated Extension States:
  **BA**
  Designated Validation States:
  **GE KH LA MA MD TN**

(30) Priority: **04.10.2024  IN 202421075240**

(71) Applicant: **Tata Consultancy Services Limited
  Maharashtra (IN)**

(72) Inventors:
  • **RAMAMURTHI, Narayanan
    600 113 Chennai, Tamil Nadu (IN)**
  • **DAS, Shyam Sundar
    700160 Kolkata, West Bengal (IN)**
  • **RANJAN, Pritish
    500081 Hyderabad, Telangana (IN)**

(74) Representative: **Goddar, Heinz J.
  Boehmert & Boehmert
  Anwaltspartnerschaft mbB
  Pettenkoferstrasse 22
  80336 München (DE)**

(54)  **SYSTEMS AND METHODS FOR GENERATING REPURPOSING DRUG  CANDIDATES FOR
  TARGET DISEASES**

(57)  Development of a new drug molecule on average requires an expenditure of US$2-3 billion and a duration of at least 13-15 years. Subsequently, repurposing the existing drugs outside the scope of the original medical indications, has attracted serious consideration. Present disclosure provides a system and method for recommending drug candidates for a target disease by generating drug-refined Medical Subject Headings (MeSH) terms frequency matrix using MeSH terms assigned to Pubmed articles. Further, topic modelling is performed on the drug-refined MeSH terms frequency matrix to generate drug-topic conditional probabilities, and drug-drug distance is computed using Kullback-Leibler divergence. The drugs are then clustered and unknown drugs of a target disease are shortlisted from a cluster with known drugs based on predicted targets and indications of unknown and known drugs. A subset of shortlisted unknown drugs is recommended as a new candidate for target disease based on supporting literature evidence.

obtaining an input data pertaining to a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD) — 202

processing the input data to obtain a list of matched drugs and a list of refined MeSH terms — 204

generating a drug-refined MeSH terms frequency matrix based on the list of matched drugs and the list of refined MeSH terms pertaining to the one or more articles — 206

performing a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs — 208

applying a Kullback–Leibler (K-L) divergence on the one or more drug-topic conditional probabilities to obtain a symmetrized pairwise drug-drug distance matrix — 210

applying one or more clustering techniques on the symmetrized pairwise drug-drug distance matrix to generate one or more drug clusters — 212

analyzing each cluster amongst the one or more drug clusters to identify at least a subset of clusters containing one or more known drugs specific to a target disease — 214

segregating a plurality of drugs of each cluster in the identified subset of clusters into a known drug group or unknown drug group — 216

Ⓐ

**FIG. 2A**

EP 4 723 129 A1

## Description

CROSS-REFERENCE TO RELATED APPLICATIONS AND PRIORITY

The present application claims priority to Indian application no. 202421075240, filed on October 04, 2024.

TECHNICAL FIELD

The disclosure herein generally relates to drug development, and, more particularly, to a system and method for generating repurposing drug candidates for target diseases.

BACKGROUND

De novo drug development is an onerous process with respect to resources. On average, development of a new drug molecule requires an expenditure of US$2-3 billion and a duration of at least 13-15 years. Subsequently, drug repurposing which is the process of finding new uses outside the scope of the original medical indications for existing drugs or compounds, has attracted serious consideration, as discovering new uses for existing drugs is time- as well as cost-efficient compared with de novo drug development. Some of the cost-intensive steps in drug development, including chemical optimization, in vitro and in vivo screening, formulation development and toxicological studies, can be eliminated if drug repurposing is considered. One of the assumptions considered for drug repurposing is that 'similar drugs' have similar therapeutic effects. To establish 'similarity' between drugs, different types of data are used, such as structures, targets and proteomic or transcriptomic networks. Once similarity is established between drugs, on the basis of any of these data types, they are explored further for treatment of similar indications.

Conventionally, a computational procedure, MeSHDD (Brown, Adam S., and Chirag J. Patel. "MeSHDD: literature-based drug-drug similarity for drug repositioning." Journal of the American Medical Informatics Association 24.3 (2017): 614-618.), was introduced which employs Medical Subject Headings (MeSH) term-based similarity between drugs to suggest a repurposing candidate. A drug-drug distance was computed using enrichment of drug-MeSH term association. Finally, k-means clustering was used to partition the drugs into clusters, from those clusters repurposing candidate was suggested for a disease based on the co-existence of the candidate drug with known drugs of that particular disease. Another literature work (Bisgin, Halil, et al. "Investigating drug repositioning opportunities in FDA drug labels through topic modeling." BMC bioinformatics. Vol. 13. BioMed Central, 2012.) used side-effects data from FDA-approved drug labels and created drug-side effects term association, performed topic modelling, computed drug-drug distance using drug-topic conditional probabilities and symmetrized K-L divergence and suggested safer alternatives of a drug using the nearest neighbor of that drug with a smaller number of associated side-effects. A third literature [Ranjan, Pritish, Shyam Sundar Das, and Narayanan Ramamurthi. "Investigation of Drug Repurposing Opportunities Using Side-effects data, Topic Modelling and Clustering Algorithms." Proceedings of the 14th ACM International Conference on Bioinformatics, Computational Biology, and Health Informatics. 2023.] proposed a procedure combining i) MeSH term refinement of Das et al. (Das, Shyam Sundar, Pritish Ranjan, and Ibrahim Roshan Kunnakkattu. "Literature-based drug-drug similarity for drug repurposing: impact of Medical Subject Headings term refinement and hierarchical clustering." Future Medicinal Chemistry 14.18 (2022): 1309-1323.), ii) drug-drug distance computation using topic modelling followed by K-L divergence of Bisgin et al. and iii) clustering of drugs by Brown et al. While the quality of drug clusters improved, the recommendation of drug candidates was still challenging. As it is not feasible to check each cluster and identify clusters with both known and unknown drugs of a target disease and then manually search appropriate literature evidence for all the unknown drugs.

SUMMARY

Embodiments of the present disclosure present technological improvements as solutions to one or more of the above-mentioned technical problems recognized by the inventors in conventional systems.

For example, in one aspect, there is provided a processor implemented method for generating repurposing drug candidates for target diseases. The method comprises obtaining, via one or more hardware processors, an input data pertaining to (i) a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD); processing, via the one or more hardware processors, the input data to obtain a list of matched drugs and a list of refined MeSH terms; generating, via the one or more hardware processors, a drug-refined MeSH terms frequency matrix based on the list of matched drugs and the list of refined MeSH terms pertaining to the one or more articles; performing, via the one or more hardware processors, a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs; applying, via the one or more hardware processors, a Kullback-Leibler (K-L) divergence on the one or more drug-topic conditional probabilities to obtain a symmetrized pairwise drug-drug distance matrix; applying, via the one or more hardware processors, one or more clustering techniques on the symmetrized pairwise drug-drug distance matrix to generate one or more drug clusters; analyzing, via the one or more hardware processors, each cluster amongst the one or more drug clusters to identify at least a subset of clusters containing one or more known drugs specific to a target disease; segregating, via the one or more hardware processors, a plurality of drugs of each cluster in the identified subset of clusters into a known drug group or unknown drug group; predicting, for each drug in the known drug group and the unknown drug group, via the one or more hardware processors, one or more targets using an associated chemical information and one or more machine learning algorithms and mapping one or more corresponding indications from the TTD to each of the predicted one or more targets; performing, via the one or more hardware processors, a comparison of the predicted one or more targets and the corresponding one or more mapped indications of (i) each drug comprised in the unknown drug group with (ii) each drug comprised in the known drug group to obtain a filtered set of unknown drugs having a similarity with one or more known drugs; identifying, via the one or more hardware processors, one or more literature documents from a literature database for each unknown drugs amongst the filtered set of unknown drugs based on (i) a list of targets relevant to the target disease obtained from the TTD, and (ii) one or more associated PubMed identifiers; and generating, via the one or more hardware processors, a set of recommended repurposing drug candidates comprising one or more unknown drugs having the similarity with the one or more known drugs of the target disease based on the one or more literature documents indicating an effectiveness against a specific target relevant to the target disease.

In an embodiment, the one or more drug clusters are generated based on a cluster number representing an associated index.

In an embodiment, the associated index optimizes a cluster stability associated with one or more drug clusters.

In another aspect, there is provided a processor implemented system for generating repurposing drug candidates for target diseases. The system comprises: a memory storing instructions; one or more communication interfaces; and one or more hardware processors coupled to the memory via the one or more communication interfaces, wherein the one or more hardware processors are configured by the instructions to obtain an input data pertaining to (i) a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD); process the input data to obtain a list of matched drugs and a list of refined MeSH terms; generate a drug-refined MeSH terms frequency matrix based on the list of matched drugs and the list of refined MeSH terms pertaining to the one or more articles; perform a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs; apply a Kullback-Leibler (K-L) divergence on the one or more drug-topic conditional probabilities to obtain a symmetrized pairwise drug-drug distance matrix; apply one or more clustering techniques on the symmetrized pairwise drug-drug distance matrix to generate one or more drug clusters; analyze each cluster amongst the one or more drug clusters to identify at least a subset of clusters containing one or more known drugs specific to a target disease; segregate a plurality of drugs of each cluster in the identified subset of clusters into a known drug group or unknown drug group; predict, for each drug in the known drug group and the unknown drug group, one or more targets using an associated chemical information and one or more machine learning algorithms and mapping one or more corresponding indications from the TTD to each of the predicted one or more targets; perform a comparison of the predicted one or more targets and the corresponding one or more mapped indications of (i) each drug comprised in the unknown drug group with (ii) each drug comprised in the known drug group to obtain a filtered set of unknown drugs having a similarity with one or more known drugs; identify one or more literature documents from a literature database for each unknown drugs amongst the filtered set of unknown drugs based on (i) a list of targets relevant to the target disease obtained from the TTD, and (ii) one or more associated PubMed identifiers; and generate a set of recommended repurposing drug candidates comprising one or more unknown drugs having the similarity with the one or more known drugs of the target disease based on the one or more literature documents indicating an effectiveness against a specific target relevant to the target disease.

In an embodiment, the one or more drug clusters are generated based on a cluster number representing an associated index.

In an embodiment, the associated index optimizes a cluster stability associated with one or more drug clusters.

In yet another aspect, there are provided one or more non-transitory machine-readable information storage mediums comprising one or more instructions which when executed by one or more hardware processors cause generating repurposing drug candidates for target diseases by obtaining an input data pertaining to (i) a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD); processing the input data to obtain a list of matched drugs and a list of refined MeSH terms; generating a drug-refined MeSH terms frequency matrix based on the list of matched drugs and the list of refined MeSH terms pertaining to the one or more articles; performing a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs; applying a Kullback-Leibler (K-L) divergence on the one or more drug-topic conditional probabilities to obtain a symmetrized pairwise drug-drug distance matrix; applying one or more clustering techniques on the symmetrized pairwise drug-drug distance matrix to generate one or more drug clusters; analyzing each cluster amongst the one or more drug clusters to identify at least a subset of clusters containing one or more known drugs specific to a target disease; segregating a plurality of drugs of each cluster in the identified subset of clusters into a known drug group or unknown drug group; predicting, for each drug in the known drug group and the unknown drug group, one or more targets using an associated chemical information and one or more machine learning algorithms and mapping one or more corresponding indications from the TTD to each of the predicted one or more targets; performing a comparison of the predicted one or more targets and the corresponding one or more mapped indications of (i) each drug comprised in the unknown drug group with (ii) each drug comprised in the known drug group to obtain a filtered set of unknown drugs having a similarity with one or more known drugs; identifying one or more literature documents from a literature database for each unknown drugs amongst the filtered set of unknown drugs based on (i) a list of targets relevant to the target disease obtained from the TTD, and (ii) one or more associated PubMed identifiers; and generating a set of recommended repurposing drug candidates comprising one or more unknown drugs having the similarity with the one or more known drugs of the target disease based on the one or more literature documents indicating an effectiveness against a specific target relevant to the target disease.

In an embodiment, the one or more drug clusters are generated based on a cluster number representing an associated index.

In an embodiment, the associated index optimizes a cluster stability associated with one or more drug clusters.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system for generating repurposing drug candidates for target diseases, in accordance with an embodiment of the present disclosure.
FIG. 2A and FIG. 2B depict an exemplary flow chart illustrating a method for generating repurposing drug candidates for target diseases, using the system of FIG. 1, in accordance with an embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

De novo drug development is an onerous process with respect to resources. On average, development of a new drug molecule requires an expenditure of US$2-3 billion and a duration of at least 13-15 years. Subsequently, drug repurposing which is the process of finding new uses outside the scope of the original medical indications for existing drugs or compounds, has attracted serious consideration, as discovering new uses for existing drugs is time- as well as cost-efficient compared with de novo drug development. Some of the cost-intensive steps in drug development, including chemical optimization, in vitro and in vivo screening, formulation development and toxicological studies, can be eliminated if drug repurposing is considered. One of the assumptions considered for drug repurposing is that 'similar drugs' have similar therapeutic effects. To establish 'similarity' between drugs, different types of data are used, such as structures, targets and proteomic or transcriptomic networks. Once similarity is established between drugs, on the basis of any of these data types, they are explored further for treatment of similar indications across various diseases.

Embodiments of the present disclosure provide system and method that process input data related to drugs and obtain a list of matched drugs and a list of refined MeSH terms. The matched drugs list and refined MeSH terms list are then used for generating drug-refined MeSH terms frequency matrix on which topic modeling is performed to obtain one or more drug-topic conditional probabilities of one or more topics comprised of one or more refined MeSH terms amongst the list of refined MeSH terms for each drug. Kullback-Leibler (K-L) divergence is then applied on the one or more drug-topic conditional probabilities to obtain a symmetrized pairwise drug-drug distance matrix on which clustering is performed to generate drug clusters. Each cluster is then analysed to identify at least a subset of clusters containing one or more known drugs specific to a target disease. Drugs of each cluster in the identified subset of clusters are segregated into a known drug group or unknown drug group. For each drug in the known drug group and the unknown drug group, one or more targets are predicted using an associated chemical information and one or more machine learning algorithms and mapped with one or more corresponding indications from the TTD. The predicted one or more targets and the corresponding one or more mapped indications of (i) each drug comprised in the unknown drug group are then compared with (ii) each drug comprised in the known drug group to obtain a filtered set of unknown drugs having a similarity with one or more known drugs based on which literature documents from a literature database are identified using (i) a list of targets relevant to the target disease obtained from the TTD, and (ii) one or more associated PubMed identifiers. A set of recommended repurposing drug candidates comprising one or more unknown drugs having the similarity with the one or more known drugs of the target disease are generated based on the one or more literature documents indicating an effectiveness against a specific target relevant to the target disease.

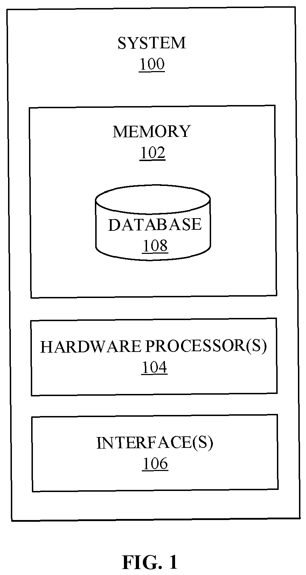

Referring now to the drawings, and more particularly to FIGS. 1 through 2B, where similar reference characters denote corresponding features consistently throughout the figures, there are shown preferred embodiments, and these embodiments are described in the context of the following exemplary system and/or method.

FIG. 1 depicts an exemplary system 100 for generating repurposing drug candidates for target diseases, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information a plurality of drugs from OpenFDA database, Medical Subject Headings (MeSH) and chemical compounds pertaining to one or more articles from Pubmed, a MeSH tree hierarchy, drug-target mapping information from ChEMBL database, a disease-target mapping information from a therapeutic targets database (TTD), and the like. The database 108 further comprises various techniques such as clustering techniques, machine learning (ML) algorithms, topic modeling, a Kullback-Leibler (K-L) divergence, and the like, which when executed by the one or more hardware processors 104 enable the system 100 to perform the method described herein. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

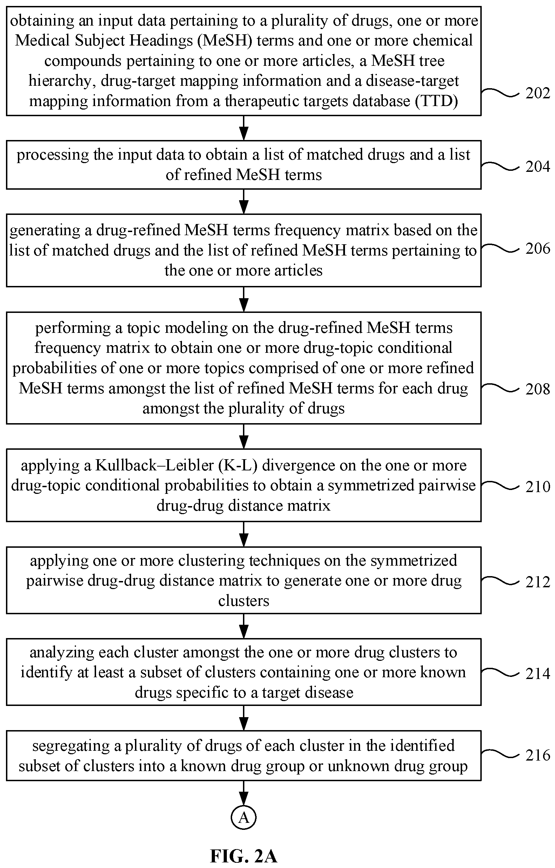

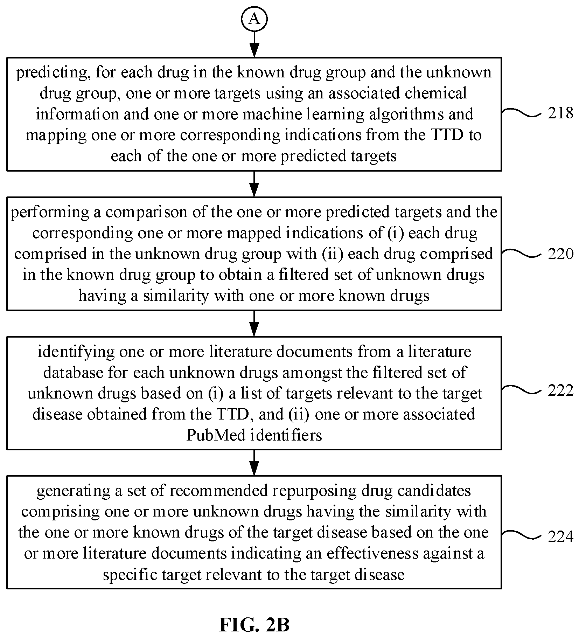

FIG. 2A and FIG. 2B, with reference to FIG. 1, depict an exemplary flow chart illustrating a method for generating repurposing drug candidates for target diseases, using the system 100 of FIG. 1, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, and the flow diagram as depicted in FIGS. 2A-2B. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

At step 202 of the method of the present disclosure, the one or more hardware processors 104 obtaining an input data pertaining to (i) a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD). For instance,

The plurality of drugs such as a list of Food and Drug Administration (FDA) approved drugs are obtained from OpenFDA (e.g., refer https://open.fda.gov/apis/drug/drugsfda/download/, date accessed: 13/10/2022). The one or more Medical Subject Headings (MeSH) terms and one or more chemical compounds pertaining to one or more articles listed in PubMed were obtained from MEDLINE Baseline Repository (MBR) (e.g., refer https://lhncbc.nlm.nih.gov/ii/information/MBR/Baselines/2021.html, date accessed: 11/10/2022). The MeSH tree Hierarchy was obtained from the National Library of Medicine (2021) (e.g., refer https://nlmpubs.nlm.nih.gov/projects/mesh/2021/meshtrees/, date accessed: 10/10/2022). The drug-target mapping information was obtained from a ChEMBL database (e.g., refer Index of /pub/databases/chembl/ChEMBLdb/latest (ebi.ac.uk), version name: ChemBL 34, date accessed: 21/05/2024). The disease-target mapping information from a therapeutic targets database (TTD) was obtained from Therapeutic Target Database (e.g., refer https://db.idrblab.net/ttd/full-data-download, date accessed: 16/02/2024).

At step 204 of the method of the present disclosure, the one or more hardware processors 104 process the input data to obtain a list of matched drugs and a list of refined MeSH terms. The above step 204 is better understood by way of following description:

MEDLINE Baseline Repository (MBR) contains various information such as PubMed ID (PMID), chemical compounds, Medical Subject Heading (MeSH) terms, abstract, author's name, etc. for each article listed in PubMed. PMID is the unique identifier of an article listed in PubMed. Chemical compounds are assigned against each of those articles based on the drugs or chemical compounds discussed in those articles. Similarly, related MeSH terms are assigned against each of these articles. The information relevant for the experiments by the present disclosure is PMID, MeSH terms and Chemical compounds. To extract the required information, the entire baseline for the year 2021 was obtained, a total of 1062 zipped folders were present in it. These contained xml files containing data pertaining to all the articles listed in PubMed. The total number of research articles, as indicated by the number of unique PMIDs was 31847923. The required information was accessed using python code and saved separately in.csv files.

To prepare a list of FDA-approved drugs which match with the chemical compounds mentioned in the Pubmed articles, the following steps were performed. The list of 2573 drugs present as active ingredients in various drug products approved by FDA was obtained from OpenFDA database. From the processed MBR, a list of all the unique chemical items (248246) was obtained. The list of chemical compounds was then compared with the list of 2573 drugs, wholly and without the suffixes (to rule out salt form variations), the results of these two matches were combined, and a list of 1799 drugs which was approved by FDA and was present in MEDLINE data, was obtained.

The MeSH tree Hierarchy was obtained for the year 2021, in one embodiment of the present disclosure. The total number of MeSH terms was 61315 with 29915 unique terms. MeSH terms were then refined to keep the classes/subclasses of MeSH terms considered to be representing mechanistic factors related to drug activity. After refinement, the total number of unique MeSH terms was 18735. The list of classes and subclasses of MeSH terms considered in the refined MeSH term list is presented in Table 1.

Table 1

| Class | Sub-class included | Sample refined MeSH terms |
|---|---|---|
| Anatomy [A] | Cells [A11] | Acinar Cells [A11.031], Allogeneic Cells [A11.047], Antibody-Producing Cells [A11.063] |
| Diseases [C] | All sub class | Carcinogenesis [C04.697.098], Cell Transformation, Neoplastic [C04.697.098.500], Blast Crisis [C04.697.098.500.110] |
| Chemicals and Drugs [D] | All sub class | Francium [D01.496.749.305.380], Polonium [D01.496.749.305.680], Promethium [D01.496.749.305.720] |
| Analytical, Diagnostic and Therapeutic Techniques and Equipment [E] | Diagnosis [E01], Therapeutics [E02] | Theranostic Nanomedicine [E01.894], Acoustic Stimulation [E02.037] |

(continued)

| Class | Sub-class included | Sample refined MeSH terms |
|---|---|---|
| Phenomena and Processes [G] | Chemical Process [G02], Metabolism [G03], Cell Physiological Phenomena [G04], Genetic Phenomena [G05], Physiological Phenomena[G07-G12] (Exclude Ocular physiological phenomena [G13] as well) | X-Ray Diffraction [G02.965], Gastric Absorption [G03.015.500.374.249], Microsatellite Instability [G05.365.590.335.590] |

At step 206 of the method of the present disclosure, the one or more hardware processors 104 generate a drug-refined MeSH terms frequency matrix based on the list of matched drugs and the list of refined MeSH terms pertaining to the one or more articles. The above step 206 is better understood by way of following description:

The MeSH terms, chemical compounds and PMID extracted from MEDLINE data were used by the system 100 for preparation of the drug-refined MeSH terms frequency matrix. Only the matched 1799 FDA-approved drugs and 18735 refined MeSH terms were considered for this. For each PMID, the refined MeSH terms and drug names associated with it were identified, and all the refined MeSH terms present in that PMID were assigned to each of the drugs present in that PMID. The association of refined MeSH terms with each drug was used to create a drug-refined MeSH terms frequency matrix for matched drugs of the size of 1799 X 18735 which represents the number of times a particular refined MeSH term was present along with a matched drug across all PMIDs. There were three columns, which contained only 0 values, those three columns had to be removed so that this dataset can be scaled correctly.

At step 208 of the method of the present disclosure, the one or more hardware processors 104 perform a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs. The above step 208 is better understood by way of following description:

To perform topic modelling using the drug-refined MeSH terms frequency matrix, the number of topics are identified by the system 100. Principal component analysis (PCA) was performed using 'prcomp' function in R [version 4.2.1] to identify the number of topics by the system 100. Since the drug-MeSH terms frequency matrix contained very diverse values ranging from 0 to 268747, PCA was performed with scaling. After performing PCA, the percentage of information for each principal component was obtained, and the cumulative information content percentage was calculated for each of them, in one embodiment of the present disclosure. For different cumulative information content percentages, the number of associated principal components were used as the topic number for further analysis by the system 100.

After the exploration of different topic numbers, 44 topics which represent 55% of information content were found to be optimal on the basis of Jaccard Index and cumulative noise cluster size and used in the subsequent steps. Jaccard Index is a metric used for understanding how stable the cluster is, as it measures the internal cluster stability of the generated clusters. The value of Jaccard Index smaller or equal to 0.5 is an indication of a "dissolved" or unstable cluster, while the value between 0.6 and 0.75, suggests the clusters may be considered as indicating patterns in the data, stable cluster should yield a Jaccard Index value of 0.75 or more (e.g., refer "Hennig, Christian, and M. A. S. S. Imports. "Package 'fpc'." Flexible procedures for clustering 1176 (2015)"). For better analysis of the clusters, any cluster with more than 80 drugs were considered as a noise cluster and those clusters have not been used for suggesting repurposing candidates. The cumulative size of all noise clusters in each of the experiments was used as one of the parameters for comparing the results produced by different experiments. Below Table 2 depicts topic number exploration using principal component analysis, by way of examples:

Table 2

| Percentage of Information | Number of Topics | Jaccard Index | Cumulative drugs in Noise Clusters | Number of Clusters |
|---|---|---|---|---|
| 50 | 32 | 0.81 | 1034 | 23 |
| 55 | 44 | 0.82 | 270 | 42 |
| 60 | 58 | 0.79 | 765 | 25 |
| 65 | 77 | 0.77 | 138 | 58 |
| 70 | 103 | 0.77 | 92 | 79 |
| 75 | 137 | 0.74 | 92 | 69 |

(continued)

| Percentage of Information | Number of Topics | Jaccard Index | Cumulative drugs in Noise Clusters | Number of Clusters |
|---|---|---|---|---|
| 90 | 384 | 0.60 | 871 | 26 |

In the present disclosure, topic modelling was performed on the drug-MeSH terms frequency matrix using the 'Tomotopy' python package [Version 0.12.5], in one embodiment of the present disclosure. This package provides implementation of multiple state-of-the-art topic modelling algorithms. It was used to run Latent Dirichlet Algorithm (LDA), Dynamic Topic Model (DTM), Correlated Topic Model (CTM) and Dirichlet Multinomial Regression (DMR). Topic modelling generated one or more drug-topic conditional probabilities of all the topics and for each drug. Each topic represents groups of refined MeSH terms. On the basis of the analysis performed by the system 100, results produced using LDA with 44 topics were found to be most suitable with a smaller number of drugs in noise clusters and high Jaccard Index value, in one embodiment of the present disclosure.

At step 210 of the method of the present disclosure, the one or more hardware processors 104 apply a Kullback-Leibler (K-L) divergence on the one or more drug-topic conditional probabilities to obtain a symmetrized pairwise drug-drug distance matrix. The above step 210 is better understood by way of following description:

The conditional topic probabilities generated using topic modelling algorithms were then used for distance computation using the Kullback-Leibler (K-L) divergence between drug-drug pairs ((e.g., refer "Bisgin, Halil, et al. "Investigating drug repositioning opportunities in FDA drug labels through topic modeling." BMC bioinformatics. Vol. 13. BioMed Central, 2012.)"). K-L divergence can be used to measure the proximity of two or more probability distributions and it is used here to compute pairwise distance between drugs using their drug-topic conditional probability distributions. However, since K-L divergence is an asymmetric measure, it was symmetrized by taking average of the unidirectional distance calculated for both ways (DrugA -> DrugB and DrugB -> DrugA). The symmetrized pairwise drug-drug distances matrix (1799 X 1799) was used as an input for clustering. Drug- topic conditional probabilities generated using LDA were used for drug-drug distance matrix computation through symmetrized KL divergence and a part of the matrix is presented in Table 3.

Table 3

| | zinc | chloroquine | oxygen | morphine | metaraminol | epinephrine | isocarboxazid |
|---|---|---|---|---|---|---|---|
| zinc | 0 | 4.032 | 6.934 | 5.857 | 11.300 | 8.109 | 6.957 |
| chloroquine | 4.032 | 0 | 5.193 | 5.240 | 7.333 | 5.046 | 4.492 |
| oxygen | 6.934 | 5.193 | 0 | 4.281 | 4.315 | 3.206 | 5.853 |
| morphine | 5.857 | 5.240 | 4.281 | 0 | 3.529 | 3.365 | 5.579 |
| metaraminol | 11.300 | 7.333 | 4.315 | 3.529 | 0 | 0.707 | 2.570 |

| | zinc | chloroquine | oxygen | morphine | metaraminol | epinephrine | isocarboxazid |
|---|---|---|---|---|---|---|---|
| epinephrine | 8.109 | 5.046 | 3.206 | 3.365 | 0.707 | 0 | 3.147 |
| isocarboxazid | 6.957 | 4.492 | 5.853 | 5.579 | 2.570 | 3.147 | 0 |

At step 212 of the method of the present disclosure, the one or more hardware processors 104 apply one or more clustering techniques on the symmetrized pairwise drug-drug distance matrix to generate one or more drug clusters. In an embodiment, the one or more drug clusters are generated based on a cluster number representing an associated index. In an embodiment, the associated index optimizes a cluster stability associated with one or more drug clusters. The above step 212 is better understood by way of following description:

Clustering is performed to group the similar drugs together. The drug-drug distance matrix was used as an input for various clustering algorithms. The clustering experiments were performed using 'clusterboot' function of 'fpc' package [Version 2.2-10]. Three different clustering algorithms were applied, k-means clustering, hierarchical clustering with Ward's linkage and Clustering Large Application (CLARA). The number of clusters was determined first by executing 100 bootstraps of cluster numbers ranging from 10 to 50 and the number which optimizes an associated index was selected as optimal cluster number. The Jaccard Index value represents the internal cluster stability, and it was used as an index to determine the optimal cluster number. Then the final drug clusters were generated using the optimal cluster number representing maximum Jaccard Index and bootstrapping with 10000 resamples. Cluster-wise drug list was accessed once the clustering was done. Drugs clusters generated using CLARA were considered for explaining subsequent computation procedure, as it had the highest Jaccard Index of 0.87 resulting in 35 clusters. Below Table 4 depicts exploration of different clustering algorithms, by way of examples:

Table 4

| Clustering Algorithm used | Jaccard Index | Noise cluster size | Number of generated clusters |
|---|---|---|---|
| CLARA | 0.8763 | 269 | 35 |
| Hierarchical clustering | 0.8254 | 270 | 42 |
| K-means clustering | 0.6922 | 677 | 27 |

At step 214 of the method of the present disclosure, the one or more hardware processors 104 analyze each cluster amongst the one or more drug clusters to identify at least a subset of clusters containing one or more known drugs specific to a target disease. The above step 214 is better understood by way of following description:

A disease is chosen for which repurposing candidate has to be suggested. The cluster-wise drug list is analyzed to identify the cluster which contains the known drugs of the target disease. To find repurposing suggestions for cancer, the clusters which contained various cancer drugs were identified, out of 35 clusters, cancer drugs were present in 5 clusters. Cluster number 13 was selected for further analysis. This cluster was used as it contained drugs for cancer as well as other drugs, while other clusters containing cancer drugs were homogeneous and did not contain drugs for any other indications.

At step 216 of the method of the present disclosure, the one or more hardware processors 104 segregate a plurality of drugs of each cluster in the identified subset of clusters into a known drug group or unknown drug group. The above step 216 is better understood by way of following description:

All the drugs of the identified cluster were partitioned into known drug group and unknown drug group for the target disease cancer based on anatomical therapeutic chemical (ATC) classification and literature evidence. The selected cluster (cluster number 13) contained a total of 47 drugs. 17 of these drugs were known to be anti-cancer drugs, while 30 drugs were not known to be anti-cancer. Below Table 5 depicts a list of drugs in known drug group and unknown drug group for the target disease cancer in cluster number 13, by way of examples:

Table 5

| List of drugs in known drugs group | List of drugs in unknown drugs group |
|---|---|
| Azacitidine, plicamycin, pentostatin, pyrvinium, masoprocol, decitabine, eflornithine, arsenic trioxide, romidepsin, bexarotene, vorinostat alitretinoin, dinutuximab, Panobinostat, selinexor cedazuridine, tretinoin | Tyrosine, nitric oxide, caffeine, dimethyl sulfoxide, adenosine, methimazole, ceruletide, acetylcysteine, trypan blue, evans blue, pentoxifylline, dinoprostone, cyclosporine, tacrolimus, rifabutin, sirolimus, becaplermin, sermorelin, amlexanox, ingenol, pegademase bovine, pirfenidone, eteplirsen, interferon gamma-1b, nusinersen, patisiran, thonzonium bromide, inotersen, viltolarsen, golodirsen |

At step 218 of the method of the present disclosure, the one or more hardware processors 104 predict, for each drug in the known drug group and the unknown drug group, one or more targets using an associated chemical information and one or more machine learning algorithms and mapping one or more corresponding indications from the TTD to each of the predicted one or more targets. The above step 218 is better understood by way of following description:

For each of the drugs in the known drug group and unknown drug group of the selected cluster, targets are predicted using morgan fingerprints of the drugs and machine learning algorithms such as logistic regression. Additionally, indications are mapped for each of them from TTD database using their predicted targets. More specifically, in the present disclosure, for each of these 17 drugs in known drug group and 30 drugs in unknown drug group, targets and indications were predicted using morgan fingerprints and logistic regression. For each of them, indications are mapped from TTD database using their predicted targets. Table 6 represents some of the predicted targets out of 722 targets considered for one unknown drug, cyclosporine, and one known drug, cedazuridine. Table 7 depicts some of the mapped indication out of 377 indications considered for one unknown drug, cyclosporine and one known drug, panobinostat.

Table 6

| Target Name | Predicted for cyclosporine | Predicted for cedazuridine |
|---|---|---|
| 11_beta_hydroxysteroid_dehydrogenase_1 | Yes | Yes |
| 11_beta_hydroxysteroid_dehydrogenase_2 | Yes | Yes |
| 5_nucleotidase | Yes | No |
| Acetylcholinesterase | Yes | No |
| Acetyl_CoA_carboxylase_1 | Yes | Yes |
| Acetyl_CoA_carboxylase_2 | Yes | Yes |
| ADAM10 | Yes | Yes |
| ADAMTS5 | Yes | Yes |
| Beta_secretase_1 | No | Yes |
| Bloom_syndrome_protein | No | Yes |

Table 7

| Indication Name | Mapped for cyclosporine | Mapped for panobinostat |
|---|---|---|
| abdominal pelvic pain [icd-11: md81] | Yes | Yes |
| abnormal micturition [icd-11: mf50] | Yes | Yes |
| abortion [icd-11: ja00] | Yes | Yes |
| b-cell lymphoma [icd-11: 2a86] | No | Yes |
| follicular lymphoma [icd-11: 2a80] | No | Yes |
| acne vulgaris [icd-11: ed80] | Yes | Yes |
| acquired cutaneous blood vessel malformation [icd-11: ef20] | Yes | Yes |
| acute diabete complication [icd-11: 5a2y] | Yes | Yes |
| henipavirus encephalitis [icd-11: 1d63] | Yes | No |
| muscular atrophy [icd-11: 8b61] | Yes | No |

At step 220 of the method of the present disclosure, the one or more hardware processors 104 perform a comparison of the predicted one or more targets and the corresponding/associated one or more mapped indications of (i) each drug comprised in the unknown drug group with (ii) each drug comprised in the known drug group to obtain a filtered set of unknown drugs having a higher similarity with one or more known drugs. The above step 220 is better understood by way of following description:

The lists of predicted targets and mapped indications for all the unknown drugs are compared with that of the each of the known drugs by calculating the Jaccard similarity between known and unknown drugs. The unknown drugs with high similarity to known drugs are selected for further analysis. Jaccard similarity, defined as $\frac{Number\ of\ common\ elements\ between\ two\ sets}{Total\ number\ of\ unique\ elements\ in\ two\ sets}$, is calculated by dividing the number of common elements between two sets with the total number of unique elements in two sets. The maximum value of Jaccard similarity observed between predicted targets of an unknown drug with any of the known drugs is stored, and the same way the maximum indication similarity is computed and stored. Finally, the maximum target and indication similarity for each unknown drug are averaged to get an average similarity score. The unknown drugs are then arranged in a descending order of their average similarity. For example, when predicted targets of an unknown drug cyclosporine were compared with that of known drugs, highest similarity was observed with cedazuridine. The number of common targets between cyclosporine and cedazuridine was 255 while the total number of unique targets predicted for both the drugs was 354, hence using the Jaccard similarity formula, the target similarity was 255/354 =0.720339. When mapped indications of an unknown drug cyclosporine were compared with that of known drugs, the highest indication similarity was observed with panobinostat. The number of common indications between cyclosporine and panobinostat was 299, while the total number of unique indications mapped for both the drugs was 320, hence the indication similarity was 299/320 = 0.934375. Thus, the average similarity was (0.720339+ 0.934375)/2 = 0.827357. Cyclosporine, an immunosuppressant which is administered to patients to prevent transplant rejection, had the fourth highest similarity among unknown drugs and was selected for further analysis, by the system 100 of the present disclosure, in one embodiment. Below Table 8 depicts a similarity of filtered set of unknown drugs with known drugs based on predicted targets and mapped indications, by way of examples:

Table 8

| Drug name | Target Similarity | Indication Similarity | Average Similarity |
| --- | --- | --- | --- |
| Nitric oxide | 0.902222 | 0.852399 | 0.877310 |
| adenosine | 0.805112 | 0.940000 | 0.872556 |
| Dimethyl sulfoxide | 0.784861 | 0.875887 | 0.830374 |
| cyclosporine | 0.720339 | 0.934375 | 0.827357 |
| methimazole | 0.748092 | 0.901235 | 0.824663 |

At step 222 of the method of the present disclosure, the one or more hardware processors 104 identify one or more literature documents from a literature database (stored in the memory 102) for each unknown drugs amongst the filtered set of unknown drugs based on (i) a list of targets relevant to the target disease obtained from the TTD, and (ii) one or more associated PubMed identifiers. The above step 222 is better understood by way of following description:

PMIDs against which the selected highly similar drug is assigned as 'Chemical compound', are identified for subsequent analysis. The PMIDs which contained Cyclosporine as a 'Chemical compound', were identified from MEDLINE 2021 dataset. There was a total of 29427 such PMIDs. Only the 500 most recent PMIDs were used for further analysis. A list of drug targets relevant to the targeted disease has been obtained from therapeutic targets database. A total of 1651 targets were found relevant to various cancers from therapeutic targets database. The system 100 then searched the relevant targets in the abstracts of the identified PMIDs of highly similar drugs. A list of PMIDs which contained the given targets was then obtained. A total of 133 out of 500 PMIDs searched contained targets relevant to various cancers. Below Table 9 depicts some of the selected targets for cancer and some of PMIDs of Cyclosporine, by way of examples:

Table 9

| PMID | Matched Target Names |
| --- | --- |
| 30897993, 30776644, 30801221, 30801551, 30739121 | t-cells, cell differentiation, matrix metalloproteinase, mmp-9, interleukin-23, interleukin-17, interleukin-2, amp-activated protein kinase, protein kinase, mineralocorticoid receptor |

At step 224 of the method of the present disclosure, the one or more hardware processors 104 generate a set of recommended repurposing drug candidates comprising one or more unknown drugs having the similarity with the one or more known drugs of the target disease based on the one or more literature documents indicating an effectiveness against a specific target relevant to the target disease. The above step 224 is better understood by way of following description:

PMIDs containing the selected targets were verified for supporting literature evidence. The articles (PMIDs) were checked if they contained information which established a favorable relationship between the considered drug and a targeted disease-relevant mechanism of action. It was observed that PMIDs related to Cyclosporine had mentions of multiple cancer targets such as 'matrix metalloprotease', 'T-cells', 'interleukin-17','amp-activated protein kinase' etc. When the PMIDs containing target names were further analysed, one of the PMIDs 30897993, contained the following text: 'Cyclosporine (CYC), a calcineurin inhibitor acts specifically on T-cells and is one of the most effective treatment options for psoriasis'. A further manual search was performed to understand the relation between T-cell and cancer, it was found that it had previously been established as a target for Acute lymphoblastic leukemia. The article also mentioned that T-cell Acute lymphoblastic leukemia could be targeted by targeting Calcineurin receptors. Another article which mentioned that Cyclosporine could target lung cancer cells through Calcineurin receptors was also found. Hence, based on the PMIDs filtered by the system 100 and additional search, Cyclosporine can be recommended as a repurposing suggestion for T-cell acute lymphoblastic leukemia. The importance of performing manual validation is reflected in the fact that the mechanism by which Cyclosporine can act against T-cell acute lymphoblastic leukemia is Calcineurin inhibition, however the target Calcineurin is not described as a target for cancer in TTD. Thus, by manual validation known targets like T-Cells can be connected with newly established targets such as Calcineurin.

Finally, the unknown drug with high similarity with respect to target and indication with known drugs of the targeted disease, is recommended as repurposing candidates by the system 100 if there is supporting literature evidence which indicates that the given drug might be effective against a certain target or process that is relevant to the targeted disease. Based on high similarity of 0.827357 with anti-cancer drugs and literature evidence, cyclosporine is suggested as repurposing candidate for cancer. The same process was repeated for other diseases as well, and 3 more repurposing candidates could be identified. Below Table 10 depicts other repurposing candidates recommended by the system 100, by way of examples:

Table 10

| Drug Name | Original Indication | Repurposed Indication |
|---|---|---|
| Tafluprost | Glaucoma | Arrhythmia |
| Teriparatide | Osteoporosis | Insulin-resistance in Diabetes |
| Aprepitant | Nausea and vomiting | Bacterial infection in CNS |

For identifying new drug candidates for a target disease based on drug-drug similarity, generally one type of information is used such as chemical information, side-effects, drug targets etc. Using information specific to only one domain may restrict the robustness of the outcome. Whereas drug-drug similarity derived from multiple data types and domains can be more robust. MeSH terms are used for indexing research articles listed in Pubmed and hence are a comprehensive source of terminology relevant to various aspects of medical research. They have an advantage over specialized databases such as SIDER and TOXNET, as they cover a wide range of topics and are not confined to a single domain of medical research. Considering all these advantages, MeSH data has been selected for computing drug-drug similarity in this disclosure.

MeSH terms are hierarchically divided into 16 classes and subsequent subclasses on the basis of subjects such as Anatomy, Organisms, Diseases, Chemical and Drugs, Health Care, Geographical etc. All the 29915 MeSH terms are not useful for establishing drug-drug similarity for finding new application of a drug. The terms which are related to mechanistic behavior of the drug and disease, can provide deeper insights and be more relevant. To specifically establish drug-drug similarity on the basis of mechanistic parameters, a set of 18735 MeSH terms from Diseases, Chemicals and Drugs etc. has been used by the system 100 and the method of the present disclosure.

MEDLINE Baseline Repository (MBR) contains MeSH terms information of 248246 chemical compounds. A list of drugs has been prepared by the present disclosure based on the chemical compounds from MBR and FDA approved drugs from OpenFDA database for efficient analysis of their possible new indications.

Computing drug-drug similarity directly from drug-refine MeSH terms frequency matrix using widely used enrichment for co-occurrence between each drug and MeSH term is not appropriate as the frequency matrix contained very diverse values ranging from 0 to 268747. Grouping the MeSH terms in relevant topics can address this problem and same has been carried out by the system 100 and the method of the present disclosure using topic modelling. For determining the number of topics for performing topic modelling, a systematic analysis has been performed based on Principal Component Analysis (PCA).

Euclidean distance measure or binary distance measure is frequently used for computing distance for real value and binary data respectively. In this case, as the distance is computed based on conditional probability distribution, use of appropriate distance measure is essential. Kullback-Leibler (K-L) divergence which is a distance measure between two probability distribution and the same is used by the system 100 and the method of the present disclosure for computing distance between two drugs using their drug-topic conditional probabilities.

Clustering of drugs has performed so that the similar drugs or drugs prescribes to treat a particular disease can be grouped in a cluster and thus finding new candidates for a target disease would be easier. Multiple clustering algorithm was employed to generate optimal and stable clusters. Jaccard Index was used to determine the cluster stability, and the size of noise clusters was defined to remove the clusters with higher number of drugs from subsequent analysis.

While the quality of drug clusters improved after using refined MeSH terms and topic modelling, the suggesting a new drug candidate for a target disease was still challenging. As it is not feasible to check each and every cluster for probable candidates and manually search appropriate literature evidence from the vast amount of literature for all the unknown drugs present with known drugs. A systematic method has been implemented/designed by the present disclosure to reduce the manual intervention and enable a system driven automation of the majority of the process. The process shortlists potential candidates based on predicted targets and mapping corresponding indication. Relevant literature was accessed by web scrapping using targets related to selected disease and PMID related to the shortlisted potential candidates. Finally, validation was carried out with filtered literature based on user inputs.

Further, the results of the system 100 and the method of the present disclosure was validated by determining the capability of the method of the present disclosure to suggest some of the drugs which has been proposed as repurposing candidates before. Itraconazole is a drug which was traditionally used for the treatment of fungal infections. However, it has been suggested as a repurposing candidate for treatment of cancer as well (Li, C. L., Fang, Z. X., Wu, Z., Hou, Y. Y., Wu, H. T., & Liu, J. (2022). Repurposed itraconazole for use in the treatment of malignancies as a promising therapeutic strategy. Biomedicine & Pharmacotherapy, 154, 113616). It has been suggested for cancer treatment because it inhibits angiogenesis, a process crucial for development of cancer (Liu, ZL., Chen, HH., Zheng, LL. et al. Angiogenic signaling pathways and anti-angiogenic therapy for cancer. Sig Transduct Target Ther 8, 198 (2023). https://doi.org/10.1038/s41392-023-01460-1). A clinical trial has also been conducted for the same (https://clinicaltrials.gov/study/NCT04481100?cond=Cancer&intr=itraconazole&sort =StudyFirstPostDate&page=2&rank=20, https://clinicaltrials.gov/study/NCT00798135). To test the effectiveness of the method of the present disclosure, the possibility of suggesting itraconazole for cancer treatment has been verified by the system 100. For that, the cluster number 35 which contained itraconazole was identified and partitioned into two groups, drugs known to treat cancer (known drug group), and drugs not known to treat cancer (unknown drug group). The cluster contained 56 drugs, out of which SMILES were not available for 5, hence they were excluded, from the remaining 51, 23 were grouped as known drugs on the basis of available literature, while the remaining 28 were grouped as unknown. The lists of all known and unknown drugs from the selected cluster are provided in below Table 11.

Table 11

| Drugs known to treat Cancer | Drugs not known to treat Cancer |
|---|---|
| pentamidine, deferasirox, flucytosine, econazole, natamycin, ketoconazole, miltefosine, acyclovir, ganciclovir, terbinafine, ciclopirox, tioconazole, naftifine, cidofovir, sertaconazole, sulconazole, terconazole, oxiconazole, posaconazole, micafungin, valganciclovir, eliglustat, brincidofovir | amphotericin b, nystatin, candicidin, miconazole, clotrimazole, foscarnet, haloprogin, hydroxystilbamidine, fluconazole, itraconazole, penciclovir, miglustat, butoconazole, butenafine, famciclovir, migalastat, valacyclovir, fomivirsen, voriconazole, anidulafungin, caspofungin, luliconazole, efinaconazole, tavaborole, ibrexafungerp, letermovir, talc, docosanol |

Once the drugs were segregated, their respective targets and indications were predicted. After prediction of targets and indications for both known and unknown drugs, comparisons were made to filter unknown drugs with highest average similarity. The five unknown drugs with highest average similarity with known drugs are mentioned in Table 12 below:

Table 12

| Drug name | Target Similarity | Indication Similarity | Average Similarity |
|---|---|---|---|
| **itraconazole** | 0.906077 | 0.990909 | 0.948493 |
| **miconazole** | 0.854938 | 0.994030 | 0.924484 |
| **penciclovir** | 0.849673 | 0.957377 | 0.903525 |
| **valacyclovir** | 0.873457 | 0.919753 | 0.896605 |
| **butoconazole** | 0.807580 | 0.973214 | 0.890397 |

Since itraconazole was seen to have the highest average similarity, it was considered for further processing. For further processing, the 500 most recent PMIDs which list itraconazole as a chemical item were obtained, a list of molecular targets related to various types of cancer was prepared using Therapeutics Targets Database (TTD). Using web-scraping and Natural Language Processing (NLP) techniques as known in the art, the abstracts of all the selected PMIDs have been checked for the presence of molecular targets relevant to cancer. A total of 72 PMIDs were found to contain names of molecular targets relevant to cancer. These PMIDs were analysed and the PMID 31965399 (Chittasupho C, Kengtrong K, Chalermnithiwong S, Sarisuta N. Anti-angiogenesis by dual action of R5K peptide conjugated itraconazole nanoparticles. AAPS PharmSciTech. 2020 Jan 21;21(3):74. doi: 10.1208/s12249-019-1568-8. PMID: 31965399.) contained names of various cancer targets like angiogenesis, vascular endothelial growth factor, vascular endothelial growth factor, etc. Also, in the abstract, it was explicitly mentioned that *"itraconazole altered the signaling pathway of VEGF stimulation"*, as it is already established that angiogenesis is a process which is crucial for cancer development which makes it important for anti-cancer therapeutic approach. VEGF (vascular endothelial growth factor) is a molecular target which plays a vital role in angiogenesis, and the fact that itraconazole has been shown to act against it provides rationale for suggesting the same as a drug candidate for cancer treatment.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method comprising:

    obtaining, via one or more hardware processors, an input data pertaining to a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD) (202);
    processing, via the one or more hardware processors, the input data to obtain a list of matched drugs and a list of refined MeSH terms (204);

generating, via the one or more hardware processors, a drug-refined MeSH terms frequency matrix based on the list of matched drugs and the list of refined MeSH terms pertaining to the one or more articles (206);

performing, via the one or more hardware processors, a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs (208);

applying, via the one or more hardware processors, a Kullback-Leibler (K-L) divergence on the one or more drug-topic conditional probabilities to obtain a symmetrized pairwise drug-drug distance matrix (210);

applying, via the one or more hardware processors, one or more clustering techniques on the symmetrized pairwise drug-drug distance matrix to generate one or more drug clusters (212);

analyzing, via the one or more hardware processors, each cluster amongst the one or more drug clusters to identify at least a subset of clusters containing one or more known drugs specific to a target disease (214);

segregating, via the one or more hardware processors, a plurality of drugs of each cluster in the identified subset of clusters into a known drug group or unknown drug group (216);

predicting, for each drug in the known drug group and the unknown drug group, via the one or more hardware processors, one or more targets using an associated chemical information and one or more machine learning algorithms and mapping one or more corresponding indications from the TTD to each of the predicted one or more targets (218);

performing, via the one or more hardware processors, a comparison of the predicted one or more targets and the corresponding one or more mapped indications of (i) each drug comprised in the unknown drug group with (ii) each drug comprised in the known drug group to obtain a filtered set of unknown drugs having a similarity with one or more known drugs (220);

identifying, via the one or more hardware processors, one or more literature documents from a literature database for each unknown drugs amongst the filtered set of unknown drugs based on (i) a list of targets relevant to the target disease obtained from the TTD, and (ii) one or more associated PubMed identifiers (222); and

generating, via the one or more hardware processors, a set of recommended repurposing drug candidates further comprising one or more unknown drugs having the similarity with the one or more known drugs of the target disease based on the one or more literature documents indicating an effectiveness against a specific target relevant to the target disease (224).

2. The processor implemented method as claimed in claim 1, wherein the one or more drug clusters are generated based on a cluster number representing an associated index.

3. The processor implemented method as claimed in claim 2, wherein the associated index optimizes a cluster stability associated with one or more drug clusters.

4. A system (100) comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

obtain an input data pertaining to a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD);

process the input data to obtain a list of matched drugs and a list of refined MeSH terms;

generate a drug-refined MeSH terms frequency matrix based on the list of matched drugs and the list of refined MeSH terms pertaining to the one or more articles;

perform a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs;

apply, a Kullback-Leibler (K-L) divergence on the one or more drug-topic conditional probabilities to obtain a symmetrized pairwise drug-drug distance matrix;

apply one or more clustering techniques on the symmetrized pairwise drug-drug distance matrix to generate one or more drug clusters;

analyze each cluster amongst the one or more drug clusters to identify at least a subset of clusters containing one or more known drugs specific to a target disease;

segregate a plurality of drugs of each cluster in the identified subset of clusters into a known drug group or unknown drug group;

predict, for each drug in the known drug group and the unknown drug group, one or more targets using an associated chemical information and one or more machine learning algorithms and mapping one or more corresponding indications from the TTD to each of the predicted one or more targets;

perform a comparison of the predicted one or more targets and the corresponding one or more mapped indications of (i) each drug comprised in the unknown drug group with (ii) each drug comprised in the known drug group to obtain a filtered set of unknown drugs having a similarity with one or more known drugs;

identify one or more literature documents from a literature database for each unknown drugs amongst the filtered set of unknown drugs based on (i) a list of targets relevant to the target disease obtained from the TTD, and (ii) one or more associated PubMed identifiers; and

generate a set of recommended repurposing drug candidates further comprising one or more unknown drugs having the similarity with the one or more known drugs of the target disease based on the one or more literature documents indicating an effectiveness against a specific target relevant to the target disease.

5. The system as claimed in claim 4, wherein the one or more drug clusters are generated based on a cluster number representing an associated index.

6. The system as claimed in claim 5, wherein the associated index optimizes a cluster stability associated with one or more drug clusters.

7. One or more non-transitory machine-readable information storage mediums comprising one or more instructions which when executed by one or more hardware processors cause:

obtaining an input data pertaining to a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD) ;

processing the input data to obtain a list of matched drugs and a list of refined MeSH terms ;

generating a drug-refined MeSH terms frequency matrix based on the list of matched drugs and the list of refined MeSH terms pertaining to the one or more articles ;

performing a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs ;

applying a Kullback-Leibler (K-L) divergence on the one or more drug-topic conditional probabilities to obtain a symmetrized pairwise drug-drug distance matrix ;

applying one or more clustering techniques on the symmetrized pairwise drug-drug distance matrix to generate one or more drug clusters ;

analyzing each cluster amongst the one or more drug clusters to identify at least a subset of clusters containing one or more known drugs specific to a target disease ;

segregating a plurality of drugs of each cluster in the identified subset of clusters into a known drug group or unknown drug group ;

predicting, for each drug in the known drug group and the unknown drug group, one or more targets using an associated chemical information and one or more machine learning algorithms and mapping one or more corresponding indications from the TTD to each of the predicted one or more targets ;

performing a comparison of the predicted one or more targets and the corresponding one or more mapped indications of (i) each drug comprised in the unknown drug group with (ii) each drug comprised in the known drug group to obtain a filtered set of unknown drugs having a similarity with one or more known drugs ;

identifying one or more literature documents from a literature database for each unknown drugs amongst the filtered set of unknown drugs based on (i) a list of targets relevant to the target disease obtained from the TTD, and (ii) one or more associated PubMed identifiers ; and

generating a set of recommended repurposing drug candidates further comprising one or more unknown drugs having the similarity with the one or more known drugs of the target disease based on the one or more literature documents indicating an effectiveness against a specific target relevant to the target disease .

8. The one or more non-transitory machine-readable information storage mediums as claimed in claim 7, wherein the one or more drug clusters are generated based on a cluster number representing an associated index.

9. The one or more non-transitory machine-readable information storage mediums as claimed in claim 8, wherein the associated index optimizes a cluster stability associated with one or more drug clusters.

SYSTEM
100

MEMORY
102

DATABASE
108

HARDWARE PROCESSOR(S)
104

INTERFACE(S)
106

**FIG. 1**

obtaining an input data pertaining to a plurality of drugs, one or more Medical Subject Headings (MeSH) terms and one or more chemical compounds pertaining to one or more articles, a MeSH tree hierarchy, drug-target mapping information and a disease-target mapping information from a therapeutic targets database (TTD) ⟶ 202

↓

processing the input data to obtain a list of matched drugs and a list of refined MeSH terms ⟶ 204

↓

generating a drug-refined MeSH terms frequency matrix based on the list of matched drugs and the list of refined MeSH terms pertaining to the one or more articles ⟶ 206

↓

performing a topic modeling on the drug-refined MeSH terms frequency matrix to obtain one or more drug-topic conditional probabilities of one or more topics comprised of one or more refined MeSH terms amongst the list of refined MeSH terms for each drug amongst the plurality of drugs ⟶ 208

↓

applying a Kullback–Leibler (K-L) divergence on the one or more drug-topic conditional probabilities to obtain a symmetrized pairwise drug-drug distance matrix ⟶ 210

↓

applying one or more clustering techniques on the symmetrized pairwise drug-drug distance matrix to generate one or more drug clusters ⟶ 212

↓

analyzing each cluster amongst the one or more drug clusters to identify at least a subset of clusters containing one or more known drugs specific to a target disease ⟶ 214

↓

segregating a plurality of drugs of each cluster in the identified subset of clusters into a known drug group or unknown drug group ⟶ 216

↓

Ⓐ

**FIG. 2A**

(A)

predicting, for each drug in the known drug group and the unknown drug group, one or more targets using an associated chemical information and one or more machine learning algorithms and mapping one or more corresponding indications from the TTD to each of the one or more predicted targets ⟿ 218

performing a comparison of the one or more predicted targets and the corresponding one or more mapped indications of (i) each drug comprised in the unknown drug group with (ii) each drug comprised in the known drug group to obtain a filtered set of unknown drugs having a similarity with one or more known drugs ⟿ 220

identifying one or more literature documents from a literature database for each unknown drugs amongst the filtered set of unknown drugs based on (i) a list of targets relevant to the target disease obtained from the TTD, and (ii) one or more associated PubMed identifiers ⟿ 222

generating a set of recommended repurposing drug candidates comprising one or more unknown drugs having the similarity with the one or more known drugs of the target disease based on the one or more literature documents indicating an effectiveness against a specific target relevant to the target disease ⟿ 224

**FIG. 2B**

# EUROPEAN SEARCH REPORT

**Application Number**

EP 25 20 1958

## DOCUMENTS CONSIDERED TO BE RELEVANT

| Category | Citation of document with indication, where appropriate, of relevant passages | Relevant to claim |
|---|---|---|
| X | SUNDAR DAS SHYAM ET AL: "Literature-Based Drug-Drug Similarity for Drug Repurposing: Impact of Medical Subject Headings Term Refinement and Hierarchical Clustering", FUTURE MEDICINAL CHEMISTRY, vol. 14, no. 18, 26 August 2022 (2022-08-26), pages 1309-1323, XP093369912, Retrieved from the Internet: URL:https://doi.org/10.4155/fmc-2022-0074> * the whole document, in particular pages 1309-1317 * | 1-9 |
| X | BROWN ADAM S ET AL: "MeSHDD: Literature-based drug-drug similarity for drug repositioning", JOURNAL OF THE AMERICAN MEDICAL INFORMATICS ASSOCIATION, vol. 24, no. 3, 27 September 2016 (2016-09-27), pages 614-618, XP093369799, AMSTERDAM, NL ISSN: 1067-5027, DOI: 10.1093/jamia/ocw142 Retrieved from the Internet: URL:http://academic.oup.com/jamia/article-pdf/24/3/614/34149163/ocw142.pdf> * the whole document * | 1-9 |

**CLASSIFICATION OF THE APPLICATION (IPC)**

INV.
G16H70/40

ADD.
G16H20/10
G16H50/50

**TECHNICAL FIELDS SEARCHED (IPC)**

G16H

The present search report has been drawn up for all claims

| Place of search | Date of completion of the search | Examiner |
|---|---|---|
| Munich | 26 February 2026 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421075240

**Non-patent literature cited in the description**

- **BROWN, ADAM S.** ; **CHIRAG J. PATEL.** MeSHDD: literature-based drug-drug similarity for drug repositioning. *Journal of the American Medical Informatics Association*, 2017, vol. 24 (3), 614-618
- Investigating drug repositioning opportunities in FDA drug labels through topic modeling. **BISGIN, HALIL et al.** BMC bioinformatics.. BioMed Central, 2012, vol. 13
- **RANJAN, PRITISH** ; **SHYAM SUNDAR DAS** ; **NARAYANAN RAMAMURTHI.** Investigation of Drug Repurposing Opportunities Using Side-effects data, Topic Modelling and Clustering Algorithms. *Proceedings of the 14th ACM International Conference on Bioinformatics, Computational Biology, and Health Informatics.*, 2023
- **DAS, SHYAM SUNDAR** ; **PRITISH RANJAN** ; **IBRAHIM ROSHAN KUNNAKKATTU.** Literature-based drug-drug similarity for drug repurposing: impact of Medical Subject Headings term refinement and hierarchical clustering.. *Future Medicinal Chemistry*, 2022, vol. 14 (18), 1309-1323
- **HENNIG, CHRISTIAN** ; **M. A. S. S.** Package 'fpc'.'' Flexible procedures for clustering. *Imports*, 2015, vol. 1176
- **LI, C. L.** ; **FANG, Z. X.** ; **WU, Z.** ; **HOU, Y. Y.** ; **WU, H. T.** ; **LIU, J.** Repurposed itraconazole for use in the treatment of malignancies as a promising therapeutic strategy.. *Biomedicine & Pharmacotherapy*, 2022, vol. 154, 113616
- **LIU, ZL.** ; **CHEN, HH.** ; **ZHENG, LL. et al.** Angiogenic signaling pathways and anti-angiogenic therapy for cancer.. *Sig Transduct Target Ther*, 2023, vol. 8, 198, https://doi.org/10.1038/s41392-023-01460-1
- **CHITTASUPHO C** ; **KENGTRONG K** ; **CHALERM-NITHIWONG S** ; **SARISUTA N.** Anti-angiogenesis by dual action of R5K peptide conjugated itraconazole nanoparticles.. *AAPS PharmSciTech.*, 21 January 2020, vol. 21 (3), 74